(19) Europäisches Patentamt / European Patent Office / Office européen des brevets

(11) **EP 4 262 548 B1**

(12) **EUROPEAN PATENT SPECIFICATION**

(45) Date of publication and mention
of the grant of the patent:
**13.08.2025 Bulletin 2025/33**

(21) Application number: **20966195.8**

(22) Date of filing: **21.12.2020**

(51) International Patent Classification (IPC):
**A61B 5/055** (2006.01)    **G01R 33/563** (2006.01)
**A61B 5/00** (2006.01)

(52) Cooperative Patent Classification (CPC):
**G01R 33/56325; A61B 5/055; A61B 5/7289;**
A61B 2576/023; G01R 33/5608; G01R 33/56366

(86) International application number:
**PCT/CA2020/051776**

(87) International publication number:
**WO 2022/133568 (30.06.2022 Gazette 2022/26)**

(54) **METHOD AND APPARATUS FOR DETERMINING BIOMARKERS OF VASCULAR FUNCTION UTILIZING BOLD CMR IMAGES**

VERFAHREN UND VORRICHTUNG ZUR BESTIMMUNG VON BIOMARKERN DER GEFÄSSFUNKTION UNTER VERWENDUNG VON BOLD CMR-BILDERN

PROCÉDÉ ET APPAREIL POUR DÉTERMINER DES BIOMARQUEURS DE FONCTION VASCULAIRE AU MOYEN D'IMAGES RMC BOLD

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.10.2023 Bulletin 2023/43**

(73) Proprietor: **Area 19 Medical Inc.**
**Montreal, Quebec H2V 2X5 (CA)**

(72) Inventors:
• **FRIEDRICH, Matthias**
**Montreal, Québec H3C 0P9 (CA)**
• **BENOVOY, Mitchel**
**Montreal, Québec H2V 2X5 (CA)**
• **HILLIER, Elizabeth Grace**
**Edmonton, Alberta T5R 5T4 (CA)**

(74) Representative: **Potter Clarkson**
**Chapel Quarter**
**Mount Street**
**Nottingham NG1 6HQ (GB)**

(56) References cited:
WO-A1-2014/022935    WO-A1-2019/220417
US-B2- 10 706 592

• "Cardiovascular Magnetic Resonance (Third Edition), A Companion to Braunwald's Heart Disease", 1 January 2019, ELSEVIER, US, ISBN: 978-0-323-41561-3, article ROHAN DHARMAKUMAR; SOTIRIOS A. TSAFTARIS; HSIN-JUNG YANG; DEBIAO LI: "Cardiovascular Magnetic Resonance Assessment of Myocardial Oxygenation", pages: 84 - 96, XP009538829, DOI: 10.1016/B978-0-323-41561-3.00009-4

• LANGHAM MICHAEL C, ENGLUND ERIN K, MOHLER EMILE R, LI CHENG, RODGERS ZACHARY B, FLOYD THOMAS F, WEHRLI FELIX W: "Quantitative CMR markers of impaired vascular reactivity associated with age and peripheral artery disease", JOURNAL OF CARDIOVASCULAR MAGNETIC RESONANCE : OFFICIAL JOURNAL OF THE SOCIETY FOR CARDIOVASCULAR MAGNETIC RESONANCE, vol. 15, no. 17, 2013, pages 1 - 10, XP021142121

EP 4 262 548 B1

## Description

## FIELD

The present disclosure relates to determining biomarkers of vascular function utilizing BOLD CMR images.

## BACKGROUND

Macrovascular and microvascular dysfunction are hallmarks of several diseases, including coronary artery disease (CAD or coronary atherosclerosis), most of them with high morbidity and mortality rates. Typically, blood supply and oxygen to the heart are affected, with consequences for longevity and quality of life. Furthermore, in the cascade of developing atherosclerosis, the deterioration of microvascular function is considered one of the first pathophysiological changes, occurring before any detectable morphological abnormalities. Thus, microvascular function is a target of choice for the early detection of atherosclerosis and other diseases affecting the heart such as diabetes, obesity, hypertension and hypercholesterolemia.

Oxygenation-sensitive cardiac magnetic resonance (CMR) using the blood oxygen-level-dependent (BOLD) effect allows for non-invasive monitoring of changes in myocardial tissue oxygenation. Oxygenation-sensitive CMR detects changes in hemoglobin oxygenation by making use of the fact that its magnetic properties change when transitioning from oxygenated to deoxygenated status. While oxygenated hemoglobin (oxyHb) is diamagnetic exhibiting a weak stabilization of the magnetic field surrounding the molecule, de-oxygenated hemoglobin (de-oxyHb) is paramagnetic, destabilizing the surrounding field and thereby leading to a loss of magnetic field homogeneity, known as the BOLD effect. CMR protocols sensitive to the BOLD effect show a regional oxygenation-sensitive signal intensity (OS-SI or BOLD-SI) drop in tissues with such a relative increase of de-oxyHb, as seen in myocardial ischemia.

Several oxygenation-sensitive approaches have been used to detect coronary artery disease, using myocardial oxygenation changes in response to vasodilation by pharmacological agents such as adenosine or dipyridamole as a marker for myocardial ischemia. While healthy vessels dilate and lead to an increase in myocardial signal intensity (SI), myocardium subtended by stenotic vessels show a blunted increase or a decrease in myocardial BOLD-SI in response to the vasodilatory trigger. However, these pharmacological agents have undesirable side effects such as bracycardia, arrhythmia, chest pain, bronchospasm, headache, nausea and heat waves. Furthermore, the injection of such vasoactive substances requires intravenous access and the availability of a medical doctor, additional cost for the vasodilatory agent, additional preparation time, and a risk for adverse events related to the injected agent. WO 2014/022935 A1 discloses an examplary use of BOLD CMR images for analysing vascular functions.

Improvements in determining biomarkers related to vascular function are desirable.

## BRIEF DESCRIPTION OF THE DRAWINGS

Embodiments of the present disclosure will now be described, by way of example only, with reference to the attached Figures.

FIG. 1 is a flow chart illustrating a method for obtaining biomarkers of microvascular or macrovascular function in an individual according to an embodiment of the present disclosure;

FIG.2 is a schematic diagram illustrating an example of performing cycle aggregation, phase-wise registration, and artifact suppression and SNR boosting of a continuously acquired BOLD CMR image series according to an embodiment of the present disclosure;

FIG. 3A to FIG. 3C is a schematic diagram illustrating an example of performing windowed singular value decomposition operation according to an embodiment of the present disclosure; and

FIG. 4 is a schematic diagram illustrating an example of performing singular value decomposition on the low-rank components of a windowed singular value operation and generating a composite image series according to an embodiment of the present disclosure.

## DETAILED DESCRIPTION

Embodiments of the present disclosure relate to a method for processing BOLD CMR image series acquired continuously, which may be referred to herein as a CINE BOLD CMR image series. The CINE BOLD CMR image series may be acquired under various periodic or non-periodic breathing states including, for example, normal breathing, controlled breathing such as for example slowed breathing or accelerated breathing, and breath hold. The method for processing CINE BOLD CMR image series may include temporally aligning CINE BOLD CMR images of CINE BOLD CMR image series at different cardiac or breathing cycles, referred to herein as cycle aggregation, and for each phase, spatially aligned the images from the cycles, referred to herein as phase-wise registration.

The method includes utilizing windowed matrix decomposition(WMD operations followed by, in some embodiments, matrix decomposition (MD) operations for phase-wise multi-artefact removal, and generating a composite image series utilizing the low-rank components generated from the WMDoperation, from which one or more biomarkers are extracted which may provide functional, tissue, or perfusion information.

The MD operations utilized in the WMD operation and the subsequent optional MD operation may each be one of a dynamic mode decomposition (DMD) operation, or a singular value decomposition (SVD) operation. In some examples, the WMD operation may utilize one of a DMD or SVD operation, and the subsequently performed MD operation may utilized the other one of a DMD or SVD operation. In some examples, performing SVD operations on CINE BOLD CMR images according to the present disclosure may provide more robust noise reduction compared to DMD operations, and therefore utilizing SVD operations may be more desirably compared to DMD operations.

By utilizing MD techniques, the present method is able to generate CINE BOLD CMR image series that have reduced or no noticeable artifacts, such as motion artifacts due to movement of the individual during image acquisition, which facilitates CINE BOLD CMR images series acquired under any breathing state, or combination of breathing states being utilized for analysis of the myocardium's perfusion via oxygen related CMR signal intensity as well as its functional and tissue characteristics at each pixel and each temporal phase of the cardiac or breathing cycle.

MD operations, including SVD and DMD operations, have conventionally been used for image decomposition in video sequences, such as, for example, decomposing a vector field of weather patterns into low-rank component showing the larger overall motion, and a high-rank component showing smaller eddy currents. Conventionally, MD operations, such as SVD and DMD, are not a technique that is applied to medical imaging analysis.

Conventionally, analysis of the heart utilizing CINE BOLD CMR images acquired during any sort of patient breathing paradigm except breath hold was hindered because the acquired images are typically misaligned with respect to each other and contaminated with severe motion artefacts due to the motion cause by the patient's breathing. Such mis-alignment and motion artefacts creates challenges to extract clinical information from such CINE BOLD CRM images.

The present disclosure provides a method for post processing CINE BOLD CMR images acquired during any breathing state, and/or acquired with or without contrast or stress agent to measure, derive, and visualize multiple clinically relevant, biomarkers resolved to respiratory and cardiac cycle.

In an embodiment, the present disclosure provides a method of obtaining biomarkers of microvascular or macrovascular function in an individual that includes receiving a continuous blood-oxygen-level-dependent (BOLD) cardiac magnetic resonance (CMR) image series spanning a plurality of cardiac cycles, cropping the received BOLD CMR image series into a plurality of single-cycle image series, each single-cycle image series spanning a single cardiac cycle, phase matching the plurality of single-cycle image series to generate a plurality of phase-matched single-cycle image series that are temporally aligned at a plurality of phases, wherein the images of each of the phase-matched single-cycle image series at a particular phase form a phase-vector, for each phase-vector, performing a Windowed Matrix Decomposition (WMD) operation in an overlapping, sliding window manner on the images of the phase-vector to generate, for each window, a low-rank image component that includes salient physiological information in the window and a high-rank image component that includes sparse information, reconstructing a plurality of noise-reduced phase-vectors utilizing the low-rank image components of each phase-vector, for each noise-reduced phase-vector, generating a composite phase image based on the images of the noise-reduced phase-vector, constructing a composite single-cycle image series composed of the generated composite phase images of the phase-vectors, and computing one or more oxygen perfusion biomarkers utilizing the composite image series.

In an example embodiment, the WMD operation is a windowed singular value decomposition (WSVD) operation.

In an example embodiment, the BOLD CMR image series comprise a Digital Imaging and Communications in Medicine (DICOM) containing timing tags, and cropping the received BOLD CMR image series comprises utilizing the timing tags to crop the BOLD CMR image series into the plurality of single-cycle image series.

In an example embodiment, cropping the received BOLD CMR image series comprises cropping the BOLD CMR image series utilizing an image-based technique to determine the plurality of cardiac cycles.

In an example embodiment, the image-based technique includes identifying diastole images of the BOLD CMR image series by comparing a relative size of a left ventricle in the images of the BOLD CMR image series and sequential image similarity metrics, and determining each single-cycle image series as the images of the BOLD CMR image series between two sequential identified diastole images.

In an example embodiment, the method further includes for each phase-vector, performing a Matrix Decomposition (MD) operation on low-rank image components generated by the WMD operation to generate, for each low-rank image component, a plurality of ranked eigen modes, and wherein reconstructing plurality of noise-reduced phase-vectors comprises reconstructing the images of the noise-reduced phase-vector utilizing a predetermined number of lowest-rank modes of the generated ranked eigen modes the most significant eigen modes.

In an example embodiment, the MD operation is a singular value decomposition (SVD) operation.

In an example embodiment, the method further includes, for each phase-vector, spatially aligning the images of the phase-vector prior to performing the WMD operation.

In an example embodiment, spatially aligning the images of the phase-vector is performed utilizing a non-rigid registration operation.

In an example embodiment, constructing a composite phase image utilizing the images of the noise-reduced phase-vector comprises performing, on the images of the noise-reduced phase-vectors, one of a two-dimensional (2D) median operation, a 2D mean operation, a principle component analysis operation, a spectral-based operation, or a machine learning based operation.

In an example embodiment, phase matching the plurality of single-cycle image series to generate the plurality of phase-matched single-cycle image series comprises generating phase-matched matrix in which the images of a given phase-matched single cycle image series are included in a respective row of the phase-matched matrix, and the columns correspond to respective phases such that the images in a given are the phase-vector for the phase that corresponds to that column.

In an example embodiment, computing one or more oxygen perfusion biomarkers utilizing the composite image series comprises segmenting each phase of the composite image series to isolate myocardial tissue to generate a segmented image series, and computing the one or more oxygen perfusion biomarkers utilizing the segmented image series.

In an example embodiment, the segmenting is performed by a machine learning system trained to isolate myocardial tissue or by manual myocardial tissue delimitation.

In an example embodiment, the one or more oxygen perfusion biomarkers include one or more of total signal intensity over time, oxygenation, deoxygenation, ratio of oxygenation to deoxygenation, differential of oxygenation to deoxygenation, oxygenation kinetics, deoxygenation kinetics, ratio of oxygenation kinetics to deoxygenation kinetics, differential of oxygenation kinetics to deoxygenation kinetics, signal intensity ratio of End-Diastolic (ED) to End-Systolic (ES) phases, differential of ED and ES, oxygen total variance, vascular function change, or vascular function change related to respiration.

In an example embodiment, receiving the continuous BOLD CMR image series comprises receiving continuous BOLD CMR image series that are obtained utilizing at least two different breathing paradigms.

In an example embodiment, the at least two different breathing paradigms are at least two of normal breathing, hyperventilation, or breath hold.

In an example embodiment, comprising computing at least one functional biomarker utilizing the composite image series, the at least one functional biomarker being at least one of radial strain, circumferential strain, ejection fraction, or systolic wall thickening.

In another embodiment, the present disclosure provides an apparatus for obtaining biomarkers of microvascular or macrovascular function in an individual that includes a processor configured to receive a continuous blood-oxygen-level-dependent (BOLD) cardiac magnetic resonance (CMR) image series spanning a plurality of cardiac cycles, crop the received BOLD CMR image series into a plurality of single-cycle image series, each single-cycle image series spanning a single cardiac cycle, phase match the plurality of single-cycle image series to generate a plurality of phase-matched single-cycle image series that are temporally aligned at a plurality of phases, wherein the images of each of the phase-matched single-cycle image series at a particular phase form a phase-vector, for each phase-vector, perform a Windowed Matrix Decomposition (WMD) operation in an overlapping, sliding window manner on the images of the phase-vector to generate, for each window, a low-rank image component that includes salient physiological information in the window and a high-rank image component that includes sparse information, reconstruct a plurality of noise-reduced phase-vectors utilizing the low-rank image components of each phase-vector, for each noise-reduced phase-vector, generate a composite phase image based on the images of the noise-reduced phase-vector, construct a composite single-cycle image series composed of the generated composite phase images of the phase-vectors, and compute one or more oxygen perfusion biomarkers utilizing the composite image series.

In an example embodiment, the WMD operation is a windowed singular value decomposition (WSVD) operation.

In an example embodiment, the BOLD CMR image series comprise a Digital Imaging and Communications in Medicine (DICOM) containing timing tags, and the processor configured to crop the received BOLD CMR image series comprises the processor configured to utilize the timing tags to crop the BOLD CMR image series into the plurality of single-cycle image series.

In an example embodiment, the processor configured to crop the received BOLD CMR image series comprises the processor configured to crop the BOLD CMR image series utilizing an image-based technique to determine the plurality of cardiac cycles.

In an example embodiment, the processor configured to utilize the image-based technique comprises the processor configured to identify diastole images of the BOLD CMR image series by comparing a relative size of a left ventricle in the images of the BOLD CMR image series and sequential image similarity metrics, and determine each single-cycle image series as the images of the BOLD CMR image series between two sequential identified diastole images.

In an example embodiment, the processor is further configured to, for each phase-vector, perform a Matrix Decomposition (MD) operation on low-rank image components generated by the WMD operation to generate, for each low-rank image component, a plurality of ranked eigen modes, and wherein the processor configured to reconstruct the plurality of noise-reduced phase-vectors comprises the processor configured to reconstruct the images of the noise-reduced phase-vector utilizing a predetermined number of lowest-rank modes of the generated ranked SVD modes the most significant eigen modes.

In an example embodiment, the MD operation is a singular value decomposition (SVD) operation.

In an example embodiment, the processor is further configured to, for each phase-vector, spatially align the images of the phase-vector prior to performing the WMD operation.

In an example embodiment, the processor configured to spatially align the images of the phase-vector comprises the processor configured to perform a non-rigid registration operation.

In an example embodiment, the processor configured to construct a composite phase image utilizing the images of the noise-reduced phase-vector comprises the processor configured to perform, on the images of the noise-reduced phase-vectors, one of a two-dimensional (2D) median operation, a 2D mean operation, a principle component analysis operation, a spectral-based operation, or a machine learning based operation.

In an example embodiment, the processor configured to phase match the plurality of single-cycle image series to generate the plurality of phase-matched single-cycle image series comprises the processor configured to generate phase-matched matrix in which the images of a given phase-matched single cycle image series are included in a respective row of the phase-matched matrix, and the columns correspond to respective phases such that the images in a given are the phase-vector for the phase that corresponds to that column.

In an example embodiment, the composite image series is a segmented image series in which each phase of the composite image series is segmented to isolate myocardial tissue, and the processor configured to compute the one or more oxygen perfusion biomarkers comprises the processor configured to utilize the segmented image series to compute the one or more oxygen perfusion biomarkers.

In an example embodiment, the segmented image series is generated by a machine learning system trained to isolate myocardial tissue or by manual myocardial tissue delimitation.

In an example embodiment, the one or more oxygen perfusion biomarkers include one or more of total signal intensity over time, oxygenation, deoxygenation, ratio of oxygenation to deoxygenation, differential of oxygenation to deoxygenation, oxygenation kinetics, deoxygenation kinetics, ratio of oxygenation kinetics to deoxygenation kinetics, differential of oxygenation kinetics to deoxygenation kinetics, signal intensity ratio of End-Diastolic (ED) to End-Systolic (ES) phases, differential of ED and ES, oxygen total variance, vascular function change, or vascular function change related to respiration.

In an example embodiment, the processor configured to receive the continuous BOLD CMR image series comprises the processor configured to receive continuous BOLD CMR image series that are obtained utilizing at least two different breathing paradigms.

In an example embodiment, wherein the at least two different breathing paradigms are at least two of normal breathing, hyperventilation, or breath hold.

In an example embodiment, the processor is further configured to compute at least one functional biomarker utilizing the composite image series, the at least one functional biomarker being at least one of radial strain, circumferential strain, ejection fraction, or systolic wall thickening.

For simplicity and clarity of illustration, reference numerals may be repeated among the figures to indicate corresponding or analogous elements. Numerous details are set forth to provide an understanding of the embodiments described herein. The embodiments may be practiced without these details. In other instances, well-known methods, procedures, and components have not been described in detail to avoid obscuring the embodiments described.

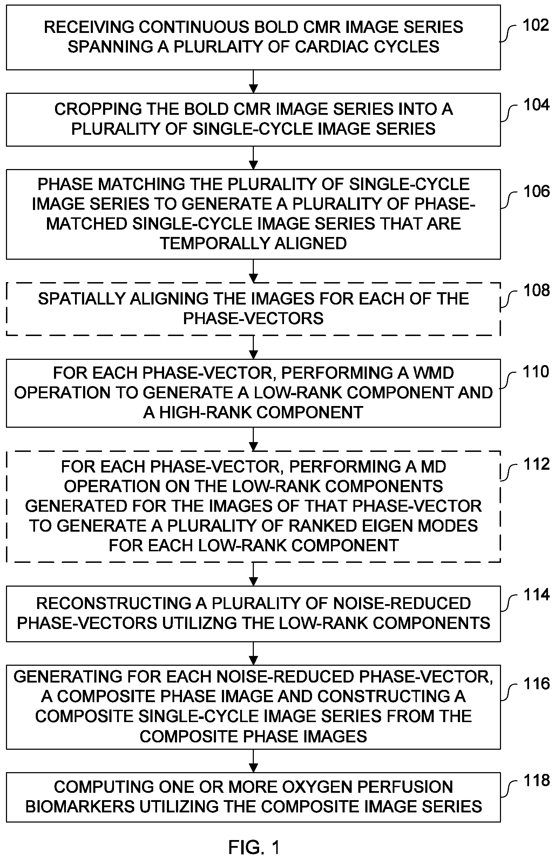

Referring to FIG. 1, a flow chart illustrating a method for obtaining biomarkers of microvascular or macro vascular function in an individual is shown. The illustrated method may be carried out by software executed, for example, by one or more processors. The processor or processors may be included within a computer or computing system comprising more than one computer, configured for processing medical images including CMR images. Coding of software for carrying out the disclosed method is within the scope of a person of ordinary skill in the art given the present description. The method may contain additional or fewer processes than shown and/or described, and may be performed in a different order. Computer-readable code executable by at least one processor to perform the process may be stored in a computer-readable storage medium, such as a non-transitory computer-readable medium.

At 102, a CINE BOLD CMR images series is received. The received CINE BOLD CMR span a plurality of cardiac cycles and may be acquired during any breathing paradigm performed by the individual, including normal breathing, breathe hold, and hyperventilation. In some examples, a portion of the received CINE BOLD CMR image series may be acquired during one breathing paradigm being performed by the individual while another portion of the received CINE BOLD CMR image series is acquired while a different breathing paradigm is performed.

In some examples, in addition to be acquired while the individual is performing one or more breathing paradigms, the CINE BOLD CMR image series may be acquired while one or more additional external stimuli are being performed on the individual. For example, the external stimuli may include one or more of a contrast-enhancing agent or a vasoactive or pharmacological stress agent being administered to the individual prior to acquiring the CINE BOLD CMR image series. Alternatively, or additionally, the additional external stimulus may be guided physical activity.

In some examples, the received CINE BOLD CMR image series may be in a Digital Imaging and Communications in Medicine (DICOM) file format. The DICOM formatting CINE BOLD CMR image series may include metadata related to the received CINE BOLD CMR image series. In an example, the metadata includes timing tags which may be utilized to identify which cardiac cycle each image of the CINE BOLD CMR image series the series pertains to. Alternatively, or additionally, the metadata may include information regarding the breathing paradigm or paradigms that the individual was instructed to perform, or what, if any, external stimuli were provided to the individual, during the acquisition of the received CINE BOLD CMR image series.

At 104, the received CINE BOLD CMR image series is cropped into a plurality of single-cycle image series, each single-cycle image series spanning a single cardiac cycle in the individual. In some examples, the received CINE BOLD CMR image series may be cropped such that each single-cycle image series spans an interval between two successive R-waves in an electrocardiogram, which may be referred to as a single "RR cycle". In some examples, the cropping performed at 104 is performed utilizing the cycle ID numbers included timing tags metadata for each of the images of a DICOM formatted CINE BOLD CMR image series, if such timing tags are present.

In examples in which timing tag metadata is not included in the received received CINE BOLD CMR image series, cropping at 104 may be performed utilizing an image based technique. In an example, the image based technique may include identifying diastole images in the received CINE BOLD CMR image series by, for example, comparing the relative size of the left ventricle using sequential image similarity metric. In this example, the images from one identified diastole image to a next sequential identified diastole image are cropped as a single-cycle image series.

At 106, the single-cycle image series from 104 are phase matched. Phase matching may be performed by, for example, temporally aligning the images of each of the single-cycle image series to generate a plurality of phase-matched single cycle images series that are temporally aligned.

In an example, the plurality of phase-matched single cycle image series are arranged in a matrix arrangement, which the single-cycle image series form the rows of the matrix, and each column of the matrix is a particular phase within the single cardiac cycle. The temporally aligned images of the single-cycle image series at a particular phase form what is referred herein as a "phase-vector".

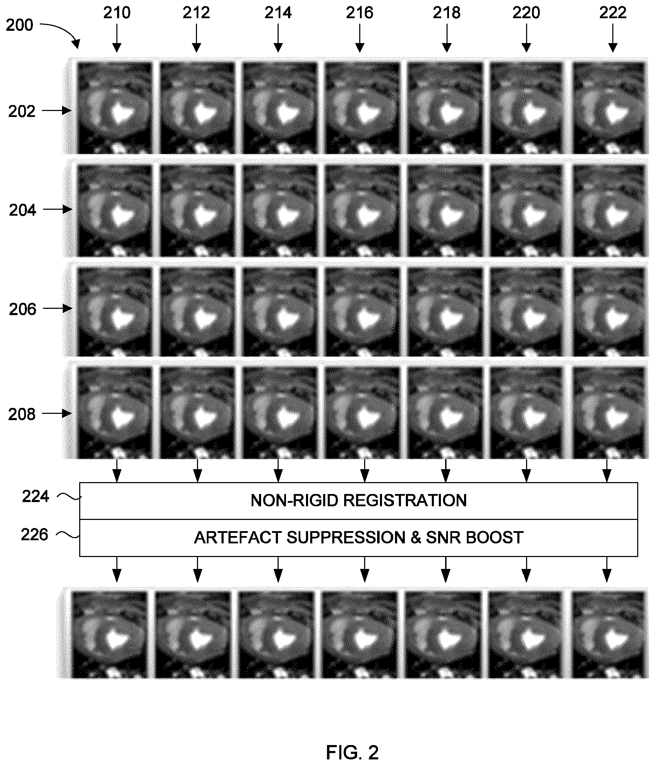

An example of such matrix arrangement is shown in FIG. 2. The matrix 200 shown in the example shown in FIG. 2 is comprised of four single-cycle image series, 202-208. Each single-cycle image series 202-208 include seven images that are temporally aligned with the images of the other single-cycle image series 202-208, forming seven phase-vectors 210-222. Although the example matrix 200 shown in FIG. 2 includes four single-cycle image series, each comprising seven phases, in general any number of single-cardiac cycle image series and any number of phases may be utilized.

Referring back to FIG. 1, optionally the images forming each phase-vector are spatially aligned with the other images of that phase-vector at 108. In an example, spatially aligning the images of each phase-vector utilizes non-rigid registration in which a geometric registration is determined that moves each point in one image to match a related point in another image. In other examples, any other suitable method for performing the optional spatially alignment of the images of each phase-vector.

Referring back to the example illustrated in FIG. 2, performing spatial alignment of the images of each of the phase-vectors 210-222 is represented by 224. Spatial alignment is performed prior to performing artefact suppression and signal-to-noise ratio (SNR) boosting, which is represented by 226. Artefact suppression and SNR boosting 226 includes performing a Windowed Matrix Decomposition (WMD) operation and may optionally include a subsequent Matrix Decomposition (MD) operation, as described in more detail below.

At 110, a WMD operation is performed overlapping, sliding window manner on the images of the phase-vector. The WMD operation includes performing a MD operation a predetermined number of images of the phase-vector, referred to as a "window", to generate a low-rank image component that includes salient physiological information of the images in the window and a high-rank image component that includes sparse information of the images in the window. Then the MD operation is performed on another window that includes a predetermined number of images of the phase-vector, at least some of the images in the new window being included in the previous window, to generate a low-rank image component and a high-rank image component for the images in the new window. This process is repeated until the window reaches the end of the phase-vector. The size of the window utilized may be determined empirically and may depend on, for example, the data on which the WMD operation is being performed.

The WMD operation performed at 110 be one of a windowed dynamic mode decomposition (WDMD) operation or a windowed singular value decomposition (WSVD) operation. In some examples, performing WSVD operations on CINE BOLD CMR images according to the present disclosure may provide more robust noise reduction compared to WDMD operations, and therefore utilizing WSVD operations may be more desirably compared to WDMD operations.

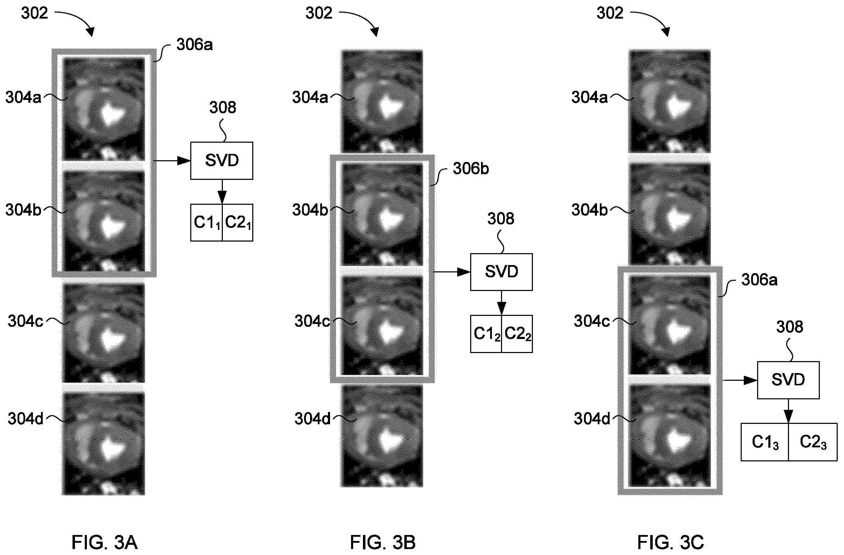

Referring to FIGS. 3A to 3C, an example of performing a WMD operation on a phase-vector 302 is shown. In the example shown, the phase-vector 302 includes four images 304a to 304d, and may be, for example, one of the phase-vectors 210-222 of the example matrix 202 shown in FIG. 2. Further, in the example shown the WMD operation is a WSVD operation that utilizes a window 306 that includes two of the images 304a-304d, however other sized windows may be utilized. In other examples, a WDMD operation may be utilized in the example shown in FIGS. 3A to 3C in place of the WSVD operation that is shown.

In FIG. 3A, a SVD operation 308 is performed on images 304a and 306b that are included in the window 306a to generate a first low-rank image component, $C1_1$ and a first high-rank image component, $C2_1$. Next, the SVD operation 308 is performed on images 304b and 306c that are included in the window 306b to generate a second low-rank image component, $C1_2$ and a second high-rank image component, $C2_2$. Then, the SVD operation 308 is performed on images 304c and 306d that are included in the window 306c to generate a third low-rank image component, $C1_3$ and a second high-rank image component, $C2_3$. In the example shown in FIGS. 3A to 3C, three SVD operations are performed to generate three low-rank image components, $C1_1$ to $C1_3$, however in general the number of SVD operations that are performed depends on the size of the window 306 and the number of images included in the phase-vector 302.

Referring back to FIG. 1, a MD operation is optionally performed on the low-rank image components generated of the WMD operations for each of the phase-vectors at 112. The optional MD operation generates, for each low-rank image component of the phase-vector, a plurality of ranked eigen modes. The MD operation that is performed on the low-rank image components may be the same MD operation that is performed on the window of images of a phase-vector. For example, the MD performed at 112 may be the SVD operation 308 described above with reference to FIGS. 3A to 3C, or may be a DMD operation.

In some examples, the WMD operation performed at 110 may utilize one of a DMD or SVD operation, and the subsequently performed MD operation at 112 may utilize the other one of a DMD or SVD operation. However, in some examples, utilizing SVD operations on CINE BOLD CMR images for both of the WMD operation performed at 110 and the MD operation performed at 112 may provide more robust noise reduction compared to utilizing a DMD operation for one or both of the WMD operation at 110 and the MD operation at 112.

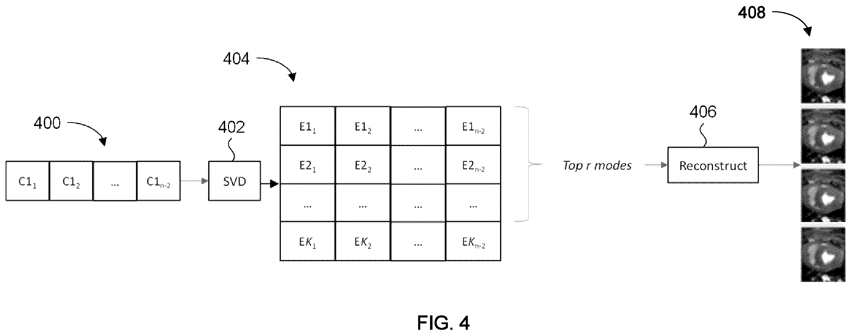

At 114, the low-rank image components are utilized to reconstruct a plurality of noise reduced phase-vectors. If the optional MD operation is performed at 112, then the low-rank image components utilized to reconstruct the plurality of noise reduced phase-vectors may be a subset of the lowest-ranked components of the ranked eigen modes generated by the MD operation, as described in more detail below with reference to the example shown in FIG. 4. Similar to the low-rank and high-rank image components generated by the WMD operation performed at 110, the lowest-ranked modes of the ranked eigen modes generated by the MD operation performed at 112 will contain most of the physiologically-relevant information, and the higher-ranked modes will contain noise and artefact information.

In examples in which the optional MD operation is not performed, then reconstructing the noise-reduced phase-vectors at 114 may be performed utilizing the low-rank image components generated by the WMD operation performed at 110.

In an example, the reconstruction of the plurality of noise reduced phase-vectors may be performed utilizing the following recomposition formula:

$$P = \Psi_{1...r}\,\omega_{1...r}^{-1}\,\Sigma\,\Lambda^{-1},$$

where $\Psi$ are the eigen modes, $\omega$ are the eigenvectors, $\Sigma$ are the singular values, and $\Lambda$ are the right singular vectors, and 1... r represents that these values are taken for the top r eigen modes. These parameters are all derived when performing low rank decomposition.

Referring to FIG. 4, an example for performing the optional MD operation at 112 to generate ranked eigen modes and reconstructing a noise-reduced phase-vector from the ranked eigen modes at 114 is illustrated. In the example shown in FIG. 4, the MD operation is a SVD operation. However, in other examples, a DMD operation may be substituted for the SVD operation.

Low-rank image components 400 generated for a particular phase-vector utilizing a WSVD operation are provided as inputs into a SVD operation 402. The low-ranked components may be generated similarly to the example described previously with reference to FIGS. 3A to 3C. In the example shown in FIG. 4, there are n low-rank image components, $C1_1$ to $C1_n$, generated by the WSVD operation performed on each phase-vector.

The number of ranks utilized may be determined empirically based on the data on which the SVD operation is performed. The number of ranks utilized may also depend on whether or not spatial alignment is performed at 108. For example, if spatial alignment is not performed at 108, fewer ranks may be desired compared to if spatial alignment was performed in order to remove more dynamic modes such that more spatial deformation modes are excluded by the SVD operation.

The SVD operation 402 is performed on each of the low-components 400, resulting in K ranked SVD modes being generated for each of the low-ranked components 400. The generated ranked SVD modes for all of the low-ranked components 400 are illustrated by the matrix 404 shown in FIG. 4, where each column in the matrix 404 corresponds to the ranked SVD modes generated for a respective one of the low-ranked image components, $C1_1$ to $C1_n$. For example, $E1_1$ to $EK_1$ are the ranked eigen components generated for the low-rank image component $C1_1$, $E1_2$ to $EK_2$ are the ranked SVD components generated for the low-rank image component $C1_2$, and so on.

Once the ranked eigen modes for all of the low-rank image components 400 are generated, a predetermined number, r, of the lowest-ranked eigen modes of the K ranked eigen modes are utilized to reconstruct 406 the noise-reduced phase-vector 408, and the other higher-ranked eigen modes are discarded.

The number of lowest-ranked eigen modes, r, may be determined empirically such that the at least a minimum amount of the dynamic information within the BOLD CMR image series is retained. Within that minimum amount of dynamic information that is retained includes the actual BOLD signal from the BOLD CMR images, which is the change of the signal caused by the underlying physiological processes. The empirical method utilized for determining the number of lowest ranked eigen modes, r, may utilize, for example, Pareto analysis.

The minimum amount of dynamic information may depend on, for example, whether the images of the phase-vector are adequately spatially aligned or not. For example, if the phase-vector images are spatially aligned at 108, then the number, r, may be determined such that, for example, 30% of the dynamic information is retained. In this case, it is assumed that physical motion of the individual during acquisition of the CINE BOLD CMR images has been removed through the spatial alignment process.

However, if spatial alignment of the images of the phase-vector is not performed, then the number, r, may be determined such that 80% of the dynamic information is retained because the actual BOLD signal is mixed with the effects of physical motion of the individual during acquisition of the CINE BOLD CMR images, and therefore a greater amount of dynamic information must be retained when spatial alignment is not performed.

After the noise-reduced phase-vector is reconstructed, a composite phase image is generated at 116. The composite phase image may be generated such that a SNR is increased. In an example, the SNR may be increased by generating the composite phase image as a two dimensional (2D) median of the images of the noise-reduced phase-vector. In other examples, different techniques may be utilized to generate the compose phase image, including for example 2D mean, principle component analysis, spectral-based methods, and machine learning based operations.

The composite phase image associated with each phase-vector are then combined together to generate a composite single-cycle image series at 116. At 118, one or more oxygen perfusion biomarkers are computed utilizing the composite single-cycle image series. Calculating the oxygen perfusion biomarkers may be performed by, for example, segmenting each phase of the composite single-cycle image series to isolate myocardial tissue, which generates a segmented image series, the computing the one or more oxygen perfusion biomarkers utilizing the segmented image series. Segmenting the images of the composite single-cycle image series may be performed by a machine learning system trained to isolate myocardial tissue or by manual myocardial tissue delimitation.

The biomarkers oxygen perfusion that are calculated at 118 may include one or more of: total signal intensity over time, which is a sum of all signal intensity values over time; oxygenation, which is a sum of increasing signal intensity values over time; deoxygenation, which is a sum of decreasing signal intensity values over time; a ratio of oxygenation to deoxygenation, which is the division of oxygen load accumulation over oxygen flush dissipation; differential of oxygenation to deoxygenation, which is the difference between oxygen load accumulation and oxygen flush dissipation; oxygenation kinetics, which may be the first order derivative, i.e., velocity, and/or the second order derivatives (i.e., acceleration) of the increasing signal intensity values over time; deoxygenation kinetics, which may be the first order derivative, i.e., velocity, and/or the second order derivatives (i.e., acceleration) of the decreasing signal intensity values over time; ratio of oxygenation kinetics to deoxygenation kinetics; differential of oxygenation kinetics to deoxygenation kinetics; signal intensity ratio of End-Diastolic (ED) to End-Systolic (ES) phases, which is the ratio of the signal intensities at ED to the signal intensities at ES; differential of ED and ES, which is the difference between the signal intensities at ED and the signal intensities at ES); oxygen total variance, which is the statistical variance of the signal intensities over time; vascular function change; or vascular function change related to respiration. These biomarkers only determinable from images acquired during active breathing. These biomarkers could not be obtained utilizing previous image processing techniques because of intrinsic noise in the imaging data acquired during active breathing.

In addition to the oxygen perfusion biomarkers computed at 118, one or more functional biomarkers may be computed utilizing the composite single-cycle image series. The functional biomarkers computed from the composite single-cycle image series may include, for example, one or more of radial strain, which is myocardial tissue displacement relative to the ventricular cavity centroid; circumferential strain, which is myocardial tissue displacement relative to the normal of the radial vector ventricular cavity centroid; ejection fraction, which is the ratio of the minimal over maximal ventricular cavity area; or systolic wall thickening, which is the difference between ED and ES wall-thicknesses.

Embodiments of the present disclosure provide processing of CINE BOLD CMR images to remove artifacts and noise, as well as increase the SNR such that CINE BOLD CMR images acquired under any breathing state, or combination of breathing states being utilized for analysis of the myocardium's perfusion. WMD operations, and in some embodiments MD operations following the WMD operations, are utilized to remove artifacts from the CINE BOLD CMR images cause by, for example, physical movement of the individual during image acquisition. Further, a composite single-cycle image series is constructed from the low-rank image components generated from the WMD operations, and in some embodiments the further MD operations, increases the SNR. In some embodiments, the artifact suppression and increase SNR is increased by spatially aligning images of each phase-vector prior to the WMD operations. By removing noise caused by, for example, physical movement of the patient by breathing during image acquisition, the above noted biomarkers can be obtained which are not obtainable utilizing previous techniques.

In the preceding description, for purposes of explanation, numerous details are set forth in order to provide a thorough understanding of the embodiments. However, it will be apparent to one skilled in the art that these specific details are not required. In other instances, well-known electrical structures and circuits are shown in block diagram form in order not to obscure the understanding. For example, specific details are not provided as to whether the embodiments described herein are implemented as a software routine, hardware circuit, firmware, or a combination thereof.

Embodiments of the disclosure can be represented as a computer program product stored in a machine-readable medium (also referred to as a computer-readable medium, a processor-readable medium, or a computer usable medium having a computer-readable program code embodied therein). The machine-readable medium can be any suitable tangible, non-transitory medium, including magnetic, optical, or electrical storage medium including a diskette, compact disk read only memory (CD-ROM), memory device (volatile or non-volatile), or similar storage mechanism. The machine-readable medium can contain various sets of instructions, code sequences, configuration information, or other data, which, when executed, cause a processor to perform steps in a method according to an embodiment of the disclosure. Those of ordinary skill in the art will appreciate that other instructions and operations necessary to implement the described implementations can also be stored on the machine-readable medium. The instructions stored on the machine-readable medium can be executed by a processor or other suitable processing device and can interface with circuitry to perform the described tasks.

The above-described embodiments are intended to be examples only. Alterations, modifications and variations can be effected to the particular embodiments by those of skill in the art without departing from the scope, which is defined solely by the claims appended hereto.

## Claims

1. Method of obtaining biomarkers of microvascular or macrovascular function in an individual comprising:

   receiving (102) a continuous blood-oxygen-level-dependent (BOLD) cardiac magnetic resonance (CMR) image series spanning a plurality of cardiac cycles;
   cropping (104) the received BOLD CMR image series into a plurality of single-cycle image series, each single-cycle image series spanning a single cardiac cycle; phase matching (106) the plurality of single-cycle image series to generate a plurality of phase-matched single-cycle image series that are temporally aligned at a plurality of phases, wherein the images of each of the phase-matched single-cycle image series at a particular phase form a phase-vector;
   for each phase-vector, performing (110) a Windowed Matrix Decomposition (WMD) operation in an overlapping, sliding window manner on the images of the phase-vector to generate, for each window, a low-rank image component that includes salient physiological information in the window and a high-rank image component that includes sparse information;
   reconstructing (114) a plurality of noise-reduced phase-vectors utilizing the low-rank image components of each phase-vector;
   for each noise-reduced phase-vector, generating (116) a composite phase image based on the images of the noise-reduced phase-vector;
   constructing a composite single-cycle image series composed of the generated composite phase images of the phase-vectors; and
   computing (118) one or more oxygen perfusion biomarkers utilizing the composite image series.

2. The method of claim 1, wherein the WMD operation is a Windowed Singular Value Decomposition (WSVD) operation.

3. The method of claim 1, wherein the BOLD CMR image series comprise a Digital Imaging and Communications in Medicine (DICOM) containing timing tags, and cropping the received BOLD CMR image series comprises utilizing the timing tags to crop the BOLD CMR image series into the plurality of single-cycle image series.

4. The method of claim 1, wherein cropping the received BOLD CMR image series comprises cropping the BOLD CMR image series utilizing an image-based technique to determine the plurality of cardiac cycles.

5. The method of claim 4, wherein the image-based technique comprises:

   identifying diastole images of the BOLD CMR image series by comparing a relative size of a left ventricle in the images of the BOLD CMR image series and sequential image similarity metrics; and

determining each single-cycle image series as the images of the BOLD CMR image series between two sequential identified diastole images.

6. The method of claim 1, further comprising:

for each phase-vector, performing a Matrix Decomposition (MD) operation on low-rank image components generated by the WMD operation to generate, for each low-rank image component, a plurality of ranked eigen modes; and wherein reconstructing plurality of noise-reduced phase-vectors comprises reconstructing the images of the noise-reduced phase-vector utilizing a predetermined number of lowest-rank modes of the generated ranked eigen modes; and

wherein optionally the MD operation is a singular value decomposition (SVD) operation.

7. The method of claim 1, further comprising, for each phase-vector, spatially aligning the images of the phase-vector prior to performing the WMD operation, and wherein spatially aligning the images of the phase-vector is optionally performed utilizing a non-rigid registration operation.

8. The method of claim 1, wherein constructing a composite phase image utilizing the images of the noise-reduced phase-vector comprises performing, on the images of the noise-reduced phase-vectors, one of:

• a two-dimensional (2D) median operation;
• a 2D mean operation;
• a principle component analysis operation;
• a spectral-based operation; or
• a machine learning based operation.

9. The method of claim 1, wherein phase matching the plurality of single-cycle image series to generate the plurality of phase-matched single-cycle image series comprises generating phase-matched matrix in which the images of a given phase-matched single cycle image series are included in a respective row of the phase-matched matrix, and the columns correspond to respective phases such that the images in a given are the phase-vector for the phase that corresponds to that column.

10. The method of claim 1, wherein computing one or more oxygen perfusion biomarkers utilizing the composite image series comprises segmenting each phase of the composite image series to isolate myocardial tissue to generate a segmented image series, and computing the one or more oxygen perfusion biomarkers utilizing the segmented image series.

11. The method of claim 10, wherein the segmenting is performed by a machine learning system trained to isolate myocardial tissue or by manual myocardial tissue delimitation.

12. The method of claim 1, wherein the one or more oxygen perfusion biomarkers include one or more of:

• total signal intensity over time;
• oxygenation;
• deoxygenation;
• ratio of oxygenation to deoxygenation;
• differential of oxygenation to deoxygenation;
• oxygenation kinetics;
• deoxygenation kinetics;
• ratio of oxygenation kinetics to deoxygenation kinetics;
• differential of oxygenation kinetics to deoxygenation kinetics;
• signal intensity ratio of End-Diastolic (ED) to End-Systolic (ES) phases;
• differential of ED and ES;
• oxygen total variance;
• vascular function change; or
• vascular function change related to respiration.

13. The method of claim 1, wherein receiving the continuous BOLD CMR image series comprises receiving continuous BOLD CMR image series that are obtained utilizing at least two different breathing paradigms, which optionally are at least two of normal breathing, hyperventilation, or breath hold.

14. The method of claim 1, further comprising computing at least one functional biomarker utilizing the composite image series, the at least one functional biomarker being at least one of:

• radial strain;
• circumferential strain;
• ejection fraction; or
• systolic wall thickening.

15. An apparatus for obtaining biomarkers of microvascular or macrovascular function in an individual comprising a processor configured to perform the method according to any one of claims 1-14.

**Patentansprüche**

1. Verfahren zum Erhalten von Biomarkern der mikrovaskulären oder makrovaskulären Funktion bei einem Individuum, umfassend:

Empfangen (102) einer kontinuierlichen, vom Blutsauerstoffspiegel abhängigen (BOLD) kardialen Magnetresonanz-(CMR)-Bildserie, die sich über mehrere Herzzyklen erstreckt;

Zuschneiden (104) der empfangenen BOLD-CMR-Bildserie in eine Vielzahl von Einzelzyklus-Bildserien, wobei jede Einzelzyklus-Bildserie einen einzelnen Herzzyklus umfasst;

Phasenabgleichen (106) der Vielzahl von Einzelzyklus-Bildserien, um eine Vielzahl von phasenabgeglichenen Einzelzyklus-Bildserien zu erzeugen, die zeitlich an einer Vielzahl von Phasen ausgerichtet sind, wobei die Bilder jeder der phasenabgeglichenen Einzelzyklus-Bildserien bei einer bestimmten Phase einen Phasenvektor bilden;

Durchführen (110) einer gefensterten Matrixzerlegungs-(WMD)-Operation für jeden Phasenvektor in einer überlappenden, gleitenden Fensterweise an den Bildern des Phasenvektors, um für jedes Fenster eine Bildkomponente mit niedrigem Rang zu erzeugen, die hervorstechende physiologische Informationen im Fenster enthält, und eine Bildkomponente mit hohem Rang, die spärliche Informationen enthält;

Rekonstruieren (114) einer Vielzahl von rauschreduzierten Phasenvektoren unter Verwendung der niedrigrangigen Bildkomponenten jedes Phasenvektors;

für jeden rauschreduzierten Phasenvektor, Erzeugen (116) eines zusammengesetzten Phasenbildes basierend auf den Bildern des rauschreduzierten Phasenvektors;

Erstellen einer zusammengesetzten Einzelzyklus-Bildserie, die aus den erzeugten zusammengesetzten Phasenbildern der Phasenvektoren besteht; und

Berechnen (118) eines oder mehrerer Sauerstoffperfusionsbiomarker unter Verwendung der zusammengesetzten Bildserie.

**2.** Verfahren nach Anspruch 1, wobei die WMD-Operation eine gefensterte Singulärwertzerlegungs-(WSVD)-Operation ist.

**3.** Verfahren nach Anspruch 1, wobei die BOLD-CMR-Bildserie eine digitale Bildgebung und Kommunikation in der Medizin (DICOM) umfasst, die Zeitmarkierungen enthält, und das Zuschneiden der empfangenen BOLD-CMR-Bildserie das Verwenden der Zeitmarkierungen zum Zuschneiden der BOLD-CMR-Bildserie in die Vielzahl von Einzelzyklus-Bildserien umfasst.

**4.** Verfahren nach Anspruch 1, wobei das Zuschneiden der empfangenen BOLD-CMR-Bildserie das Zuschneiden der BOLD-CMR-Bildserie unter Verwendung einer bildbasierten Technik umfasst, um die Vielzahl der Herzzyklen zu bestimmen.

**5.** Verfahren nach Anspruch 4, wobei die bildbasierte Technik Folgendes umfasst:

Identifizieren von Diastolenbildern der BOLD-CMR-Bildserie durch Vergleichen einer relativen Größe eines linken Ventrikels in den Bildern der BOLD-CMR-Bildserie und sequenzieller Bildähnlichkeitsmetriken; und

Bestimmen jeder Einzelzyklus-Bildserie als die Bilder der BOLD-CMR-Bildserie zwischen zwei aufeinanderfolgenden identifizierten Diastolenbildern.

**6.** Verfahren nach Anspruch 1, ferner umfassend:

Durchführen einer Matrixzerlegungs-(MD)-Operation für jeden Phasenvektor an den durch die WMD-Operation erzeugten niederrangigen Bildkomponenten, um für jede niederrangige Bildkomponente eine Vielzahl von geordneten Eigenmoden zu erzeugen; und

wobei das Rekonstruieren einer Vielzahl von rauschreduzierten Phasenvektoren das Rekonstruieren der Bilder des rauschreduzierten Phasenvektors unter Verwendung einer vorgegebenen Anzahl von Moden mit dem niedrigsten Rang der erzeugten Eigenmoden mit Rang umfasst; und

wobei die MD-Operation optional eine Singulärwertzerlegungs-(SVD)-Operation ist.

**7.** Verfahren nach Anspruch 1, das ferner für jeden Phasenvektor das räumliche Ausrichten der Bilder des Phasenvektors vor dem Durchführen der WMD-Operation umfasst, und wobei das räumliche Ausrichten der Bilder des Phasenvektors optional unter Verwendung einer nicht starren Registrierungsoperation durchgeführt wird.

**8.** Verfahren nach Anspruch 1, wobei das Erstellen eines zusammengesetzten Phasenbildes unter Verwendung der Bilder des rauschreduzierten Phasenvektors das Durchführen einer der folgenden an den Bildern der rauschreduzierten Phasenvektoren umfasst:

• eine zweidimensionale (2D) Medianoperation;
• eine 2D-Mittelwertoperation;
• eine Hauptkomponentenanalyse;
• eine spektralbasierte Operation; oder
• eine auf maschinellem Lernen basierende Operation.

**9.** Verfahren nach Anspruch 1, wobei die Phasenanpassung der Vielzahl von Einzelzyklus-Bildserien zum Erzeugen der Vielzahl von phasenangepassten Einzelzyklus-Bildserien das Erzeugen einer phasenangepassten Matrix umfasst, in der die Bilder einer gegebenen phasenangepassten Einzelzyklus-Bildserie in einer jeweiligen Zeile der phasenangepassten Matrix enthalten sind und die Spalten jeweiligen Phasen entsprechen, sodass die Bilder in einer gegebenen Zeile der Phasenvektor für die Phase sind, die dieser Spalte entspricht.

10. Verfahren nach Anspruch 1, wobei das Berechnen eines oder mehrerer Sauerstoffperfusionsbiomarker unter Verwendung der zusammengesetzten Bildserie das Segmentieren jeder Phase der zusammengesetzten Bildserie, um Myokardgewebe zu isolieren und eine segmentierte Bildserie zu erzeugen, und das Berechnen des einen oder der mehreren Sauerstoffperfusionsbiomarker unter Verwendung der segmentierten Bildserie umfasst.

11. Verfahren nach Anspruch 10, wobei die Segmentierung durch ein maschinelles Lernsystem, das darauf trainiert ist, Myokardgewebe zu isolieren, oder durch manuelle Abgrenzung von Myokardgewebe durchgeführt wird.

12. Verfahren nach Anspruch 1, wobei der eine oder die mehreren Sauerstoffperfusionsbiomarker einen oder mehrere der folgenden umfassen:

   • Gesamtsignalintensität im Zeitverlauf;
   • Sauerstoffsättigung;
   • Desoxygenierung;
   • Verhältnis von Sauerstoffsättigung zu Desoxygenierung;
   • Differenz von Sauerstoffsättigung zu Desoxygenierung;
   • Sauerstoffsättigungskinetik;
   • Desoxygenierungskinetik;
   • Verhältnis von Sauerstoffsättigungskinetik zu Desoxygenierungskinetik;
   • Differenz von Sauerstoffsättigungskinetik zu Desoxygenierungskinetik;
   • Signalintensitätsverhältnis der enddiastolischen (ED) zur endsystolischen (ES) Phase;
   • Differenz von ED und ES;
   • Gesamtsauerstoffvarianz;
   • Veränderung der Gefäßfunktion; oder
   • Veränderung der Gefäßfunktion im Zusammenhang mit der Atmung.

13. Verfahren nach Anspruch 1, wobei das Empfangen der kontinuierlichen BOLD-CMR-Bildserie das Empfangen kontinuierlicher BOLD-CMR-Bildserien umfasst, die unter Verwendung von mindestens zwei unterschiedlichen Atmungsparadigmen gewonnen werden, bei denen es sich wahlweise um mindestens zwei der folgenden handelt: normale Atmung, Hyperventilation oder Atemanhalten.

14. Verfahren nach Anspruch 1, ferner umfassend das Berechnen mindestens eines funktionellen Biomarkers unter Verwendung der zusammengesetzten Bildserie, wobei der mindestens eine funktionelle Biomarker mindestens einer der folgenden ist:

   • radiale Dehnung;
   • zirkuläre Dehnung;
   • Auswurffraktion; oder
   • systolische Wandverdickung.

15. Vorrichtung zum Erhalten von Biomarkern der mikrovaskulären oder makrovaskulären Funktion bei einem Individuum, die einen Prozessor umfasst, der zum Durchführen des Verfahrens nach einem der Ansprüche 1 bis 14 konfiguriert ist.

**Revendications**

1. Procédé d'obtention de biomarqueurs de fonction microvasculaire ou macrovasculaire chez un individu comprenant :

   la réception (102) d'une série d'images continue de résonance magnétique cardiaque (CMR) dépendante du niveau d'oxygène dans le sang (BOLD) couvrant une pluralité de cycles cardiaques ;
   le découpage (104) de la série **d'images** CMR BOLD reçue en une pluralité de séries d'images à cycle unique, chaque série **d'images** à cycle unique couvrant un seul cycle cardiaque ;
   la mise en correspondance de phase (106) de la pluralité de séries d'images à cycle unique pour générer une pluralité de séries d'images à cycle unique à phase mise en correspondance qui est alignée temporellement à une pluralité de phases, dans lequel les images de chacune des séries d'images à cycle unique à phase mise en correspondance à une phase particulière forment un vecteur de phase ;
   pour chaque vecteur de phase, la réalisation (110) d'une opération de décomposition matricielle fenêtrée (WMD) de manière superposée et coulissante sur les images du vecteur de phase pour générer, pour chaque fenêtre, un composant d'image de rang bas qui comporte des informations physiologiques importantes dans la fenêtre et un composant d'image de rang élevé qui comporte des informations éparses ;
   la reconstruction (114) d'une pluralité de vecteurs de phase à bruit réduit en utilisant les composants d'image de rang bas de chaque vecteur de phase ;
   pour chaque vecteur de phase à bruit réduit, la génération (116) d'une image de phase composite sur la base des images du vecteur de phase à bruit réduit ;

la construction d'une série d'images composites à cycle unique composée des images de phase composites générées des vecteurs de phase ; et le calcul (118) d'un ou plusieurs biomarqueurs de perfusion d'oxygène en utilisant la série d'images composites.

2. Procédé selon la revendication 1, dans lequel l'opération WMD est une opération de décomposition fenêtrée en valeurs singulières (WSVD).

3. Procédé selon la revendication 1, dans lequel les séries d'images CMR BOLD comprennent des balises de synchronisation contenant une imagerie numérique et des communications en médecine (DICOM), et le découpage de la série d'images CMR BOLD reçue comprend l'utilisation des balises de synchronisation pour découper la série d'images CMR BOLD en la pluralité de séries d'images à cycle unique.

4. Procédé selon la revendication 1, dans lequel le découpage de la série d'images CMR BOLD reçue comprend le découpage de la série d'images CMR BOLD en utilisant une technique basée sur l'image pour déterminer la pluralité de cycles cardiaques.

5. Procédé selon la revendication 4, dans lequel la technique basée sur l'image comprend :

l'identification d'images de diastole de la série d'images CMR BOLD en comparant une taille relative d'un ventricule gauche dans les images de la série d'images CMR BOLD et des mesures de similarité d'images séquentielles ; et la détermination de chaque série d'images à cycle unique comme étant les images de la série d'images CMR BOLD entre deux images de diastole séquentielles identifiées.

6. Procédé selon la revendication 1, comprenant en outre :

pour chaque vecteur de phase, la réalisation d'une opération de décomposition matricielle (MD) sur des composants d'image de rang bas générés par l'opération WMD pour générer, pour chaque composant d'image de rang bas, une pluralité de modes propres classés ; et dans lequel la reconstruction d'une pluralité de vecteurs de phase à bruit réduit comprend la reconstruction des images du vecteur de phase à bruit réduit en utilisant un nombre prédéterminé de modes de rang le plus bas des modes propres classés générés ; et dans lequel, éventuellement, l'opération MD est une opération de décomposition en valeurs singulières (SVD).

7. Procédé selon la revendication 1, comprenant en outre, pour chaque vecteur de phase, l'alignement spatial des images du vecteur de phase avant la réalisation de l'opération WMD, et dans lequel l'alignement spatial des images du vecteur de phase est éventuellement réalisé en utilisant une opération d'enregistrement non rigide.

8. Procédé selon la revendication 1, dans lequel la construction d'une image de phase composite en utilisant les images du vecteur de phase à bruit réduit comprend la réalisation, sur les images des vecteurs de phase à bruit réduit, de l'une :

• d'une opération de médiane bidimensionnelle (2D) ;
• d'une opération de moyenne 2D ;
• d'une opération d'analyse en composantes principales ;
• d'une opération basée sur le spectre ; ou
• d'une opération basée sur l'apprentissage automatique.

9. Procédé selon la revendication 1, dans lequel la mise en correspondance de phase de la pluralité de séries d'images à cycle unique pour générer la pluralité de séries d'images à cycle unique à phase mise en correspondance comprend la génération d'une matrice à phase mise en correspondance dans laquelle les images d'une série d'images à cycle unique à phase mise en correspondance donnée sont incluses dans une ligne respective de la matrice à phase mise en correspondance, et les colonnes correspondent à des phases respectives de sorte que les images dans une donnée sont le vecteur de phase pour la phase qui correspond à cette colonne.

10. Procédé selon la revendication 1, dans lequel le calcul d'un ou de plusieurs biomarqueurs de perfusion d'oxygène en utilisant la série d'images composites comprend la segmentation de chaque phase de la série d'images composites pour isoler un tissu myocardique afin de générer une série d'images segmentées, et le calcul des un ou plusieurs biomarqueurs de perfusion d'oxygène en utilisant la série d'images segmentées.

11. Procédé selon la revendication 10, dans lequel la segmentation est réalisée par un système d'apprentissage automatique entraîné pour isoler un tissu myocardique ou par délimitation manuelle de tissu myocardique.

12. Procédé selon la revendication 1, dans lequel les un ou plusieurs biomarqueurs de perfusion d'oxygène comportent un ou plusieurs :

> • d'une intensité totale de signal au fil du temps ;
> • d'une oxygénation ;
> • d'une désoxygénation ;
> • d'un rapport entre l'oxygénation et la désoxygénation ;
> • d'un différentiel entre l'oxygénation et la désoxygénation ;
> • d'une cinétique d'oxygénation ;
> • d'une cinétique de désoxygénation ;
> • d'un rapport entre une cinétique d'oxygénation et une cinétique de désoxygénation ;
> • d'un différentiel entre une cinétique d'oxygénation et une cinétique de désoxygénation ;
> • d'un rapport d'intensité de signal entre la phase de fin de diastole (ED) et la phase de fin de systole (ES) ;
> • d'un différentiel entre ED et ES ;
> • d'une variance totale de l'oxygène ;
> • d'une modification de la fonction vasculaire ; ou
> • d'une modification de la fonction vasculaire liée à la respiration.

13. Procédé selon la revendication 1, dans lequel la réception de la série d'images CMR BOLD continue comprend la réception de séries d'images CMR BOLD continues qui sont obtenues en utilisant au moins deux paradigmes de respiration différents, qui sont éventuellement au moins deux parmi la respiration normale, l'hyperventilation ou l'apnée.

14. Procédé selon la revendication 1, comprenant en outre le calcul d'au moins un biomarqueur fonctionnel en utilisant la série d'images composites, l'au moins un biomarqueur fonctionnel étant au moins l'un :

> • d'une déformation radiale ;
> • d'une déformation circonférentielle ;
> • d'une fraction d'éjection ; ou
> • d'un épaississement de la paroi systolique.

15. Appareil permettant d'obtenir des biomarqueurs de fonction microvasculaire ou macrovasculaire chez un individu comprenant un processeur configuré pour réaliser le procédé selon l'une quelconque des revendications 1 à 14.

RECEIVING CONTINUOUS BOLD CMR IMAGE SERIES SPANNING A PLURLAITY OF CARDIAC CYCLES — 102

CROPPING THE BOLD CMR IMAGE SERIES INTO A PLURALITY OF SINGLE-CYCLE IMAGE SERIES — 104

PHASE MATCHING THE PLURALITY OF SINGLE-CYCLE IMAGE SERIES TO GENERATE A PLURALITY OF PHASE-MATCHED SINGLE-CYCLE IMAGE SERIES THAT ARE TEMPORALLY ALIGNED — 106

SPATIALLY ALIGNING THE IMAGES FOR EACH OF THE PHASE-VECTORS — 108

FOR EACH PHASE-VECTOR, PERFORMING A WMD OPERATION TO GENERATE A LOW-RANK COMPONENT AND A HIGH-RANK COMPONENT — 110

FOR EACH PHASE-VECTOR, PERFORMING A MD OPERATION ON THE LOW-RANK COMPONENTS GENERATED FOR THE IMAGES OF THAT PHASE-VECTOR TO GENERATE A PLURALITY OF RANKED EIGEN MODES FOR EACH LOW-RANK COMPONENT — 112

RECONSTRUCTING A PLURALITY OF NOISE-REDUCED PHASE-VECTORS UTILIZNG THE LOW-RANK COMPONENTS — 114

GENERATING FOR EACH NOISE-REDUCED PHASE-VECTOR, A COMPOSITE PHASE IMAGE AND CONSTRUCTING A COMPOSITE SINGLE-CYCLE IMAGE SERIES FROM THE COMPOSITE PHASE IMAGES — 116

COMPUTING ONE OR MORE OXYGEN PERFUSION BIOMARKERS UTILIZING THE COMPOSITE IMAGE SERIES — 118

FIG. 1

FIG. 2

FIG. 3A          FIG. 3B          FIG. 3C

400

$C1_1$ | $C1_2$ | ... | $C1_{n-2}$

402

SVD

404

| $E1_1$ | $E1_2$ | ... | $E1_{n-2}$ |
| $E2_1$ | $E2_2$ | ... | $E2_{n-2}$ |
| ... | ... | ... | ... |
| $EK_1$ | $EK_2$ | ... | $EK_{n-2}$ |

*Top r modes*

406

Reconstruct

408

FIG. 4

**REFERENCES CITED IN THE DESCRIPTION**

**Patent documents cited in the description**

- WO 2014022935 A1